# EUROPEAN PATENT SPECIFICATION

(11) **EP 3 984 574 B1**
(45) Date of publication and mention of the grant of the patent: **23.04.2025**
(21) Application number: 20201811.5
(22) Date of filing: 14.10.2020
(51) Int. Cl.: A61M 5/315, A61M 5/31

(54) **SYRINGE WITH COMPRESSIBLE SPACER**
SPRITZE MIT KOMPRIMIERBAREM ABSTANDSHALTER
SERINGUE AYANT UN ESPACEUR COMPRESSIBLE

(43) Date of publication of application: 20.04.2022
(73) Proprietor: Gerresheimer Bünde GmbH, 32257 Bünde (DE)
(72) Inventor: Zeiß, Bernd, 49324 Melle (DE)
(74) Representative: Fish & Richardson P.C.

(56) References cited:
- US-A1- 2010 311 177
- US-A1- 2014 350 483
- US-A1- 2016 095 604

## Description

### TECHNICAL FIELD

This disclosure relates to syringes for administering liquids into the body.

### BACKGROUND

Generally, syringes are used to inject liquids (e.g. liquid medicines or saline solution) into the body or to extract blood from the body. Syringes include a barrel, a gasket or stopper that forms an air-tight and liquid-tight seal with the barrel, and a piston or plunger that translates the stopper. The barrel includes a tip that is fitted with a hollow hypodermic needle. Syringe barrels can be made of various materials, such as metal, glass, or polymers (plastics).

Some syringes are pre-filled with a single dose of medication. Pre-filled syringes are used to both package and deliver the medication and face certain requirements. For example, the material of the syringe barrel must be free from impurities, so-called extractables and leachables, that affect the stability or efficacy of the medication stored in the barrel. The material of the barrel should also prevent oxygen permeation and form a tight seal with the gasket during storage. High-performance plastics, such as cyclic olefin polymers (COP), can be used to manufacture the barrel of a pre-filled syringe.

Other syringes are manually filled, e.g., from a vial containing a liquid medicine.

Syringes face strict safety and manufacturing requirements, for example, with respect to the materials used for the syringe components. The specific medication administered using a syringe can introduce further requirements, for example, the speed at which the stopper and plunger are moved in order to dispense the medication.

US 2014/350483 A1 discloses a syringe containing a compressible porous matrix, which compressible porous matrix has in it a pharmaceutical in a soluble glass, methods of producing and using the syringe, and compressible porous matrix inserts for insertion into a syringe barrel.

### SUMMARY

The present invention provides a syringe, a syringe barrel and a method of filling a syringe that can accurately and safely administer liquid. In particular, claim 1 provides such a syringe barrel, claim 5 provides such a syringe, and claim 7 provides a such a method of filling a syringe. Further advantageous embodiments of the present invention are set out in the dependent claims.

According to the present invnention, a syringe barrel includes a hollow body with an opening configured to receive a stopper, a tip configured for connection to a hollow needle, and a compressible spacer arranged inside the hollow body and adjacent to the tip, wherein the spacer comprises an insoluble material configured to displace a liquid filled in the hollow body.

There is a gap between the compressible spacer and the inner surface of the barrel. The compressible spacer is configured to displace but not to absorb or retain the liquid inside of the barrel.

According to the present disclosure, the spacer includes a porous material configured to absorb the liquid.

According to the present disclosure, the porous material includes a foam, in particular a metal, latex, rubber, polymer, or alginate foam.

According to the present disclosure, the porous material includes a polyurethane, polystyrene, or polyolefin foam.

Preferably, the syringe barrel includes a neck portion arranged between the hollow body and the tip. The spacer includes a protrusion, and the neck portion is configured to receive the protrusion.

Preferably, the hollow body is sized for a 0.5 ml, a 1.0 ml, or a 1.5 ml syringe, and the spacer is configured to displace 10 to 100 µl, in particular 10 to 50 µl of a liquid.

Preferably, the spacer consists of a single piece of material.

According to the present invention, a syringe including a syringe barrel according to any of the embodiments above and a stopper received in the body of the syringe barrel is provided. The spacer is arranged between the barrel tip and the stopper.

Preferably, the hollow body includes an annular inner shoulder, wherein the spacer rests against the annular inner shoulder, and an end face of the stopper abuts the spacer.

According to the present invention, a method of filling a syringe includes: arranging a syringe barrel in a holder with an open end of the syringe barrel pointing upwards; inserting a compressible spacer that includes an insoluble material configured to displace a liquid into the syringe barrel through the open end; filling the syringe barrel with the liquid, in particular a liquid pharmaceutical composition, through the open end; and inserting a stopper into the syringe barrel through the open end. There is a gap between the compressible spacer and the inner surface of the barrel. The compressible spacer is configured to displace but not to absorb or retain the liquid inside of the barrel.

According to the present disclosure, the spacer includes a porous material configured to absorb the liquid.

Preferably, the liquid pharmaceutical composition includes vascular endothelial growth factor (VEGF) inhibitors.

Preferably, the syringe barrel is sized for a 0.5 ml, a 1.0 ml, or a 1.5 ml syringe, and filling the syringe barrel with a liquid includes filling 10 µl to 100 µl of the liquid into the syringe barrel.

Preferably, inserting the spacer into the syringe barrel through the open end includes: arranging a tube with one end inside of the syringe barrel; inserting the spacer into an opposite end of the tube; and pushing, using a plunger, the spacer through the tube and into the syringe barrel.

Preferably, the syringe barrel is one of a plurality of syringe barrels arranged in a tray, with the open end of each syringe barrel pointing upwards, and a compressible spacer is inserted into each syringe barrel in the tray. The implementation further includes sealing the tray for storage before filling each syringe barrel with the liquid.

Preferably, the method includes using a filling and vent tube stoppering machine to fill multiple syringe barrels with the liquid and insert stoppers into multiple syringe barrels in parallel.

The details of one or more implementations of the subject matter of this specification are set forth in the accompanying drawings and the subsequent description. Other features, aspects, and advantages of the subject matter will become apparent from the description, the drawings, and the claims.

### DESCRIPTION OF THE DRAWINGS

FIG. 1 is a schematic cross-sectional view of a pre-filled syringe.
FIG. 2 is a schematic cross-sectional view of a syringe and a compressible spacer according to an implementation.
FIG. 3 is an enlarged schematic representation of a compressible spacer according to an implementation.
FIG. 4 is a schematic cross-sectional view of a pre-filled syringe according to an implementation.
FIG. 5 is a schematic cross-sectional view of the syringe of FIG. 4 attached to a hypodermic needle.
FIG. 6 is an enlarged, partial cross-section of a syringe according to two implementations.
FIG. 7 is a flowchart for filling a syringe according to an implementation

Like reference numbers represent corresponding parts throughout the disclosure.

### DETAILED DESCRIPTION

This disclosure relates to syringes for injecting liquids in the body. Injected liquids can serve a therapeutic purpose and include pharmaceutical compositions in some cases. Other examples of injected liquids are saline solution. The following description is not limited with respect to the injected liquid and therefore uses the generic term "liquid."

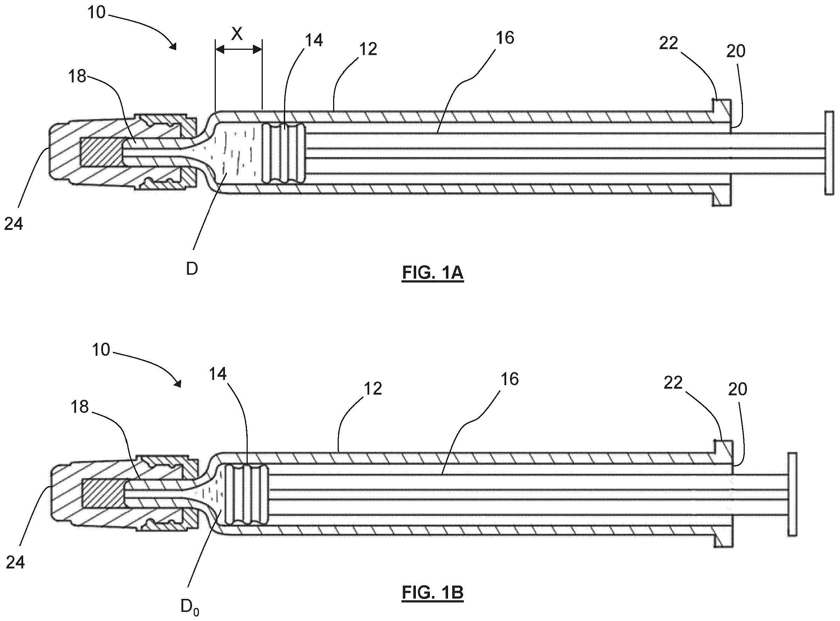

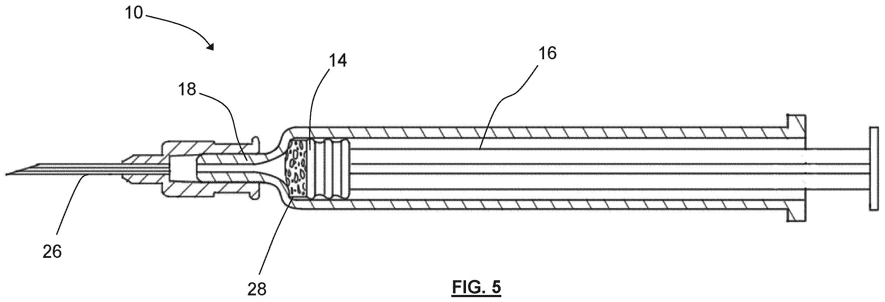

Referring to **FIG. 1A**, a syringe 10 is used to introduce liquids, into skin or tissue and includes a barrel 12, a gasket or stopper 14 that seals the barrel 12, and a piston or plunger 16 that is connected to the stopper 14. The barrel 12 is a continuous structure that extends from a tip 18 to an opening 20. A flange 22 can be provided adjacent to the opening.. In the illustrated embodiment, the tip 18 of the barrel 12 is covered by a cap 24, e.g., a Luer lock-style connector. Prior to use, the cap 24 can be replaced by a hollow needle 26 (FIG. 5). In some embodiments, a needle is integrally molded with the tip 18. The liquid is dispensed by holding the flange 22 and depressing the plunger 16. The plunger 16 can be stored separately from the stopper 14 and connected prior to use.

Generally speaking, syringe barrels are molded, coated, and assembled with a cap in automated systems. Several empty barrels are loaded, tip first, into a tray or nest with the open end of the barrel exposed. Such trays can be loaded into filling and stoppering machines that inject the liquid substance into the barrel and insert the stopper through the opening of the barrel to manufacture a pre-filled syringe. When the stopper is inserted into the barrel, it is aligned with the level of the liquid. Depending on the amount or dose of substance, the stopper will have a different position along the length of the barrel.

Referring again to FIG. 1A, the barrel 12 contains a particular dosage D of a liquid medicine, and the stopper 14 is arranged at the liquid level of the dosage D. The dosage D is small relative to the total capacity of the barrel 12, as shown by the empty space behind the stopper 14. An even smaller dosage D₀ in the same barrel 12 is shown in **FIG. 1B**. As the dosage D becomes progressively smaller, a distance X between the stopper 14 and the tip 18 decreases. As the distance X decreases, it becomes difficult to tell whether the syringe 10 is filled, especially for colorless liquid medicines. The drugs also become more difficult to dispense in a smooth manner.

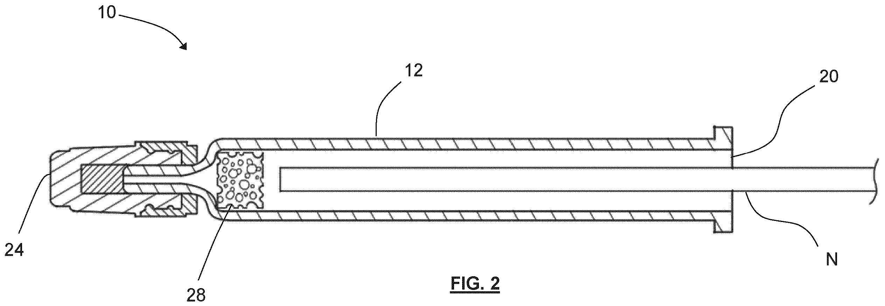

In some cases, a different size of barrel 12 can be used to accommodate small dosages D₀ and maintain a certain distance X between the stopper 14 and the tip 18. However, using different barrel sizes increases the complexity of manufacturing, filling, and stoppering syringes. For example, a given filling and stoppering machine may not be able to accommodate all possible barrel sizes. Such issues particularly arise for dosages in the microliter range, e.g., 10 to 100 µl, since the capacity of the most common syringes is 0.5ml or 1 ml. In some cases, capacity of the syringe ranges from 0.5 ml to 3 ml. Dosages of liquid medicines in this capacity are used, e.g., in ophthalmological treatments or in gene cell therapies. Example implementations of a standard syringe (e.g. a 0.5 ml or 1 ml syringe) that is adapted to dosages in the microliter range are shown in FIG. 2 to 6.

**In** **FIG. 2****,** a compressible spacer 28 inserted in the barrel 12 prior to filling. For example, the spacer 28 can be inserted using an insertion rod or tube that is similar to the equipment used to insert a stopper 14. The spacer 28 is sized and shaped so that it can be inserted through the opening 20 of the barrel 12. As explained below in more detail, the spacer 28 is made of an elastic or compressible material. In some cases, the spacer 28 can include a porous material. In this disclosure, "porous" means that the spacer 28 has many small holes. In some implementations, liquid medicine can pass through the holes in the spacer's porous material.

For example, a nozzle N of a syringe filling machine can enter the barrel 12 through the opening and fill the barrel 12 with a predetermined quantity or dosage D₀ of a liquid medicine (not shown). The liquid medicine can pass through the pores within spacer 28 and come to rest in the space between the spacer 28 and, e.g., the cap 24. In such cases according to the present disclosure, the spacer's pores retain and absorb the liquid medicine, while the rest of the spacer displaces the liquid medicine. As described below, according to the present invention, the spacer displaces the liquid medicine without absorbing it.

The nozzle N can be part of an existing filling machine or system that is used to fill other types of syringes. Automated filling machines can be programmed to precisely fill a syringe with a pre-determined dosage of liquid medicine, which is more hygienic and accurate than, e.g., manually filling a syringe from a vial of liquid to a marking on the syringe barrel. The improvement in accuracy becomes more pronounced as the size of the dosage decreases. Additionally, automated filling machines can help to reduce drug-related waste can arise, e.g., when some of the drug remains in a vial after a syringe is manually filled.

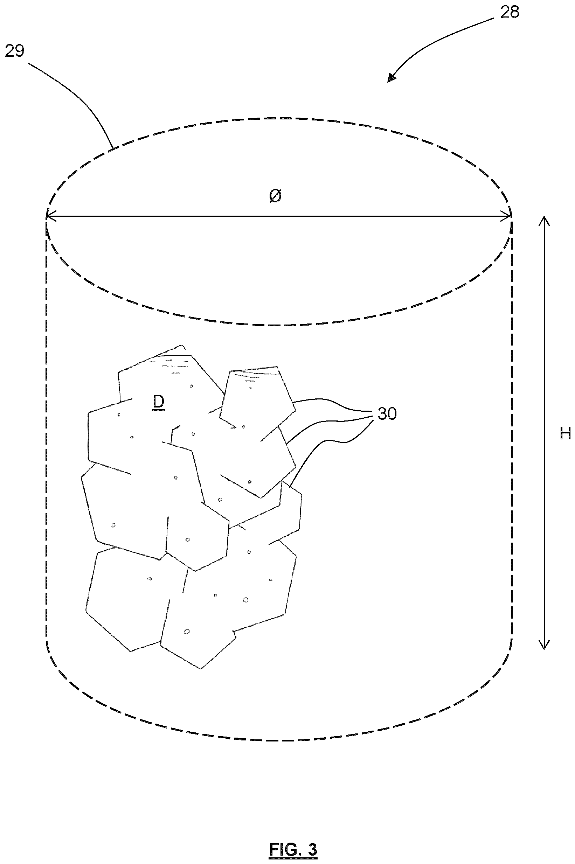

In **FIG. 3**, a spacer 28 is shown in a relaxed or uncompressed state that is also shown in FIG. 2. In this state, the spacer 28 is not subjected to external forces and has a size or volume that can be expressed, e.g., in terms of diameter Ø and height H. In this disclosure, "compressible" means that the size or volume of the spacer 28 can be reduced by applying pressure, e.g., from the stopper 14 and the walls of the barrel 12. Although the spacer 28 is shown having a cylindrical shape 29, it can also take the shape of a rectangular or hexagonal block, for example. In FIG. 3, the spacer 28 is schematically illustrated as having a continuous monolithic shape 29. For example, the spacer 28 can consist of a single piece of material cut from a larger piece of material. However, the spacer 28 can also include multiple parts. For example, the spacer 28 can include an elastic compressible material with a coated or laminated outer surface.

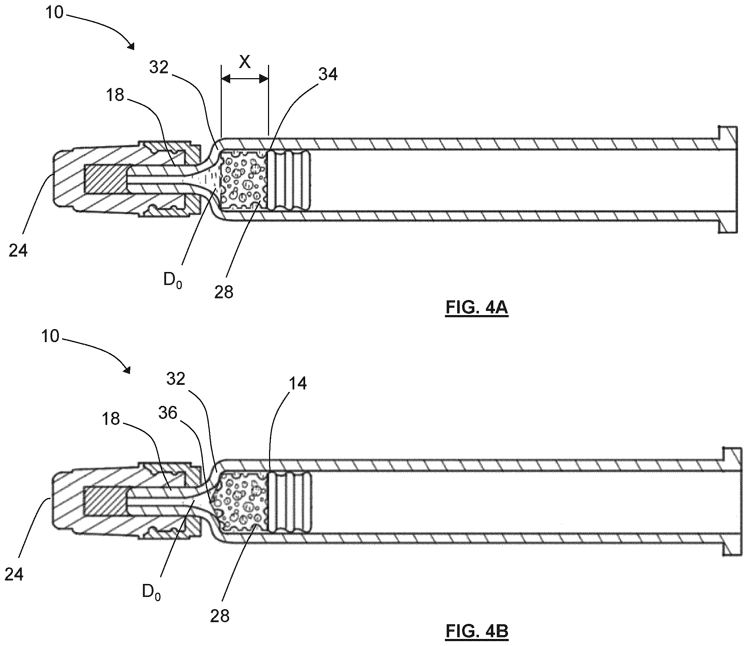

The spacer 28 can include a porous material, meaning that it has a structure that includes a plurality of interconnected cells 30 or pores, as schematically shown in FIG. 4. More specifically, each cell 30 has walls, but the walls of the cells are not completely closed. Such an open-cell structure allows the liquid medicine to pass through and fill the spacer, similarly to a sponge absorbing water. In this disclosure, "absorb" means that the material of spacer 28 can temporarily retain the liquid medicine within its pores or cells, and the liquid medicine can be expelled from the pores or cell by applying pressure to the spacer 28, e.g., with the stopper 14 and the plunger 16. Furthermore, the material of the spacer is "insoluble," meaning that it is incapable of being dissolved in in the liquid medicine.

Although all of the cells 30 in FIG. 3 are shown having open walls, the material of the spacer can include a mixture of open and closed cells 30. The structure of the spacer 28 can also depend on the material used for the spacer 28. For example, the spacer 28 can include a porous material that comprises a foam, in particular a metal, latex, rubber, polymer, or alginate foam. Polymer foams can include polyurethane, polystyrene, or polyolefin foams. In some implementations, the material of the spacer can be low-particle material that meets the standards of USP <789> and/or meet the biocompatibility standards of ISO 10993. Other criteria for selecting the spacer's material can include compatibility with the liquid medicine, shelf stability, toxicity, extractables and leachables, material elasticity, or material compressibility.

Each material can have a particular density or porosity. The choice of such properties can be adjusted to the dosage D₀ of liquid medicine. For example, material of the spacer can be chosen so that the total volume of the pores or cells 30 is based on the dosage D₀, e.g., 20, 25, 30, 40, 50, or 100 µl. Once part of the dosage D₀ has been absorbed by the spacer 28, a stopper 14 is inserted to form a pre-filled syringe, as illustrated in **FIG. 4A**. Due to the size of the spacer 28, a sufficient distance X between the stopper 14 and the barrel tip 18 is maintained despite the small volume of dosage D₀. For example, the same filling and stoppering machine that is used for syringes without a spacer can also be used for the syringe 10 in FIG. 4.

In FIG. 4A, a first end of the spacer 28 rests against an annular inner shoulder 32 of the barrel 12 that is adjacent to the tip 18. A second end of the spacer 28 abuts an end face 34 of the stopper 14. In this case, the height H of the spacer 28 substantially corresponds to the distance X between the stopper 14 and the tip 18. In some cases, the height H of the spacer 28 can be chosen to provide a certain distance X or stroke length of the stopper 14 and piston 16 as the liquid is dispensed. Additionally or alternatively, the diameter Ø of the spacer 28 may substantially correspond to the inner diameter of the syringe barrel 12. In this context, "substantially correspond" can include a gap between the spacer 28 and the inner surface of the barrel 12 that makes it easier to insert the spacer 28 during assembly.

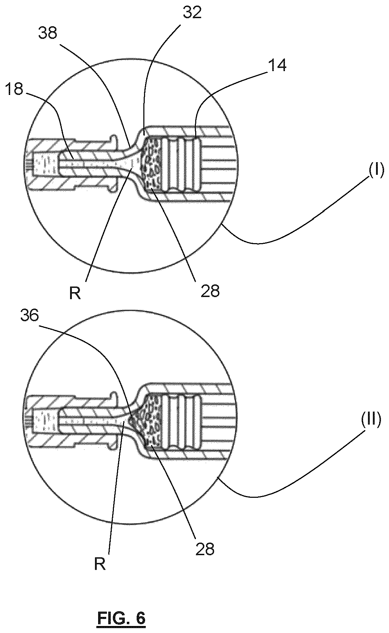

The syringe 10 and spacer 28 shown in FIG. 4B are similar to the ones shown in FIG. 4A. However, the spacer 28 includes a projection or protrusion 36 that extends past the inner shoulder 32 and toward the tip 18 of the barrel 12. The projection 36 is described in more detail below in reference to FIG. 6.

**In** **FIG. 5****,** the cap 24 is replaced by a hollow needle 26 that is attached to the barrel tip 18 for injecting the liquid medicine. The drug is injected by moving the plunger 16 and stopper 14 towards the barrel tip 18. This movement compresses the spacer 28 and reduces its size relative to what is shown in FIG. 2 to 4. When a certain level of compression is reached, the spacer 28 prevents the stopper 14 from moving any further towards the barrel tip 18. The deformation characteristics of the spacer 28 can provide haptic feedback to the user that the intended dosage D₀ has been discharged from the syringe. Overall, the described syringe 10 may reduce the risk of overdosing that can occur when multiple amounts of the intended microliter dosage are filled in a syringe, and the user must manually control the amount of liquid that is injected into the patient. Such overfilling also leads to increased cost and waste of the pharmacological product. In some cases, the density of the spacer material can be used to estimate an appropriately sized spacer for a particular dosage. Tests, measurements, and pre-clinical trials can be conducted to determine the specific dimensions to ensure an exact dosage of liquid.

In some cases, the spacer's material can be selected to provide a particular mechanical response or deformation. For example, a material can be selected so that pushing the plunger 16 and the stopper 14 causes plastic deformation of the spacer 28. In this disclosure, "plastic deformation" refers to a permanent deformation or change in the spacer's shape, i.e., the spacer 28 does not fully or partially return to its initial size and shape. Alternatively, the spacer may elastically deform, i.e., regain at least part of its initial shape after compression. In such cases, a delay between the compression and re-expansion can prevent the stopper 14 from moving away from the tip 18 and creating a vacuum in the barrel 12 after injection has been completed.

FIG. 6 illustrates compressed views of the spacers in FIG. 4A and 4B. More specifically, an upper view (I) corresponds to an enlarged version of FIG. 5, showing the spacer 28 of FIG. 4A in the compressed state. As the stopper 14 presses the spacer 28 against the annular inner shoulder 32 of the barrel, the end surface may slightly bulge towards the tip 18 without extending past the inner shoulder 32. In such cases, a neck portion 38 between the inner shoulder 32 and the tip 18 of the barrel 12 may still hold a residual volume R of the liquid. In contrast to this, the lower view (II) shows the spacer 28 of FIG. 4B, which includes the protrusion 36. At the time of compression, the protrusion 36 extends into the neck portion 38 of the barrel 12 and can reduce the residual volume R of liquid that remains in the neck portion 38. Such a reduction may be advantageous for small doses (e.g. microliters) of costly medications. In FIG. 4B and 6, the protrusion 36 is shown as having a tapered or conical shapes. However, other shapes that are adapted to the neck portion 38 of the barrel 12 can also be used. Furthermore, although the protrusion 36 is shown in conjunction with a cylindrical spacer 28, spacers having other shapes may also include a protrusion 36.

As described in FIG. 2 to 6, the spacer 28 occupies a predetermined portion of the syringe barrel 12 to more comfortably accommodate small dosages of liquid medicines. The spacer 28 also increases the stroke length of the stopper 14 and plunger 16 (distance X) for injecting the small dosage of liquid medicines, making it possible to gently inject smaller dosages into the patient. For example, dosages of liquid medicines in the range of microliters are injected into the human eye in the treatment of macular degeneration.

In addition to adapting the inner volume of a standard syringe, the spacer described above also resists the movement of the stopper 14 towards the tip 18. In some cases, a low-viscosity liquid may require a slow injection speed. One way to slow the injection speed is to increase friction between the outer surface of the stopper 14 and the inner surface of the barrel 12, i.e., increase the glide force. However, the spacer 28 can also be used to augment increased friction between the stopper and the barrel or to counteract low friction between the stopper and barrel.

This effect of the spacer 28 is not limited to dosages of liquid in the microliter range. For example, the material of the spacer 28 can be selected based on the compression characteristics to provide a "braking effect" that slows injection. One material property that can influence resistance or this braking effect is the stiffness of the material. In such cases according to the present invention, the spacer 28 may include an elastic compressible materials with closed pores that do not absorb or retain the liquid. In other words, spacers according to the present invention displace but do not absorb the liquid inside of the barrel. The spacer 28 can also be laminated or include some kind of coating to prevent absorption of the liquid. In such implementations, the likelihood of liquid remaining in the spacer after injection is reduced.

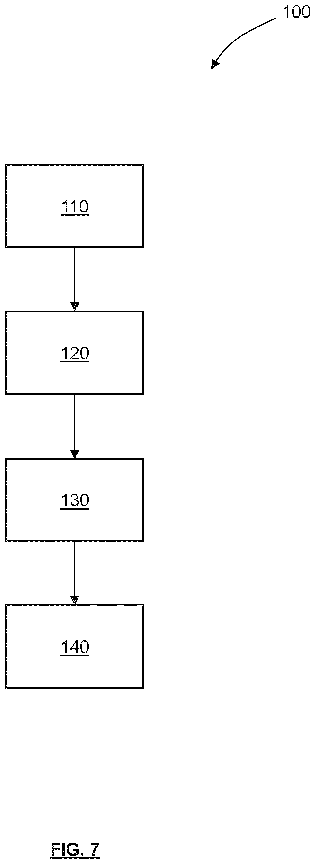

Referring to **FIG. 7**, a method 100 of filling a syringe 10 is schematically shown. The method 100 comprises: arranging 110 a syringe barrel in a holder with an open end of the syringe barrel pointing upwards; inserting 120 a compressible spacer configured to absorb a liquid into the syringe barrel through the open end; filling 130 the syringe barrel with the liquid through the open end; and inserting 140 a stopper into the syringe barrel through the open end.

In some implementations of the method 100, the liquid comprises a liquid pharmaceutical composition, such as vascular endothelial growth factor (VEGF) inhibitors that inhibit the activity of VEGF. VEGF stimulate blood vessel formation. VEGF-A plays a role in wet form age-related macular degeneration. VEGF inhibitors can be administered in the form of intravitreal injections.

In some implementations of the method 100, the syringe barrel is sized for a 0.5 ml, a 1.0 ml, a 1.5 ml, 2.0 ml, 2.5 ml, or a 3.0 ml syringe, and filling 130 the syringe barrel with a liquid comprises filling 10 µl to 100 µl, more specifically 10 µl to 50 µl, and in particular, 10 µl, 20 µl, 30 µl, 40 µl, or 50 µl, of the liquid into the syringe barrel. In this way, the method 100 can use standard syringe sizes to accurately administer microliter dosages of liquids in an ergonomic manner, as described above. The present disclosure includes all combinations of syringe barrel sizes and dosages of liquids described above, e.g. a barrel sized for a 1.5 ml syringe and 50 µl of the liquid. The disclosure also includes ranges between the specific points within the list, e.g. a barrel size ranging from 1.0 to 2.0 ml combined with a dosage ranging from 20 to 50 µl.

In some implementations of the method 100, inserting 120 the spacer into the syringe barrel through the open end comprises the use of vent tube stoppering, in other words, the tube used to insert the stopper 14 into the syringe 10. For example, using a positioning tube can include arranging a positioning tube with one end inside of the syringe barrel 12; inserting the spacer into an opposite end of the positioning tube; and pushing, using a plunger, the spacer through the positioning tube and into the syringe barrel.

Although the method 100 can be implemented to fill a single syringe 10, it can also be used to fill a tray or nest that contains multiple identical syringes. In such implementations, the syringe barrel 12 is one of a plurality of syringe barrels arranged in a tray, with the open end of each syringe barrel pointing upwards. Trays of syringe barrels 12 can be sealed after manufacturing to maintain hygiene and safety standards. In such cases, inserting 120 the spacer into each syringe barrel can happen before or after the sealing and packaging step. When it takes place before the sealing step, the tray can be moved at a later time to, e.g., a filling and vent tube stoppering station for filling the syringe barrels and inserting the stoppers.

In implementations that include the use of a filling and vent tube stoppering machine, one part of the machine can fill one syringe barrel with the liquid while another part of the machine simultaneously inserts a stopper into a barrel that has already been filled. This type of parallel processing may reduce the overall manufacturing time for filling the syringe. In such implementations, it is also possible for a third part of the machine to simultaneously insert a compressible spacer in yet further syringe barrel.

The implementations above have been described for pre-filled syringes. Generally speaking, pre-filled syringes are limited to a small number of formats or volumes, and the machines and equipment for filling pre-filled syringes are based on the size and volume of the syringe. Under these circumstances, the implementations of the present disclosure can adapt existing syringes to very small injection volumes, as described above. However, the implementations are not limited to pre-filled syringes and can also be applied to disposable, single-use syringes. For example, single-use syringes are manually filled from a shelf-stable vial of liquid.

While this specification contains many specific details of implementations, these should not be construed as limitations on the scope of any invention or of what may be claimed, but rather as descriptions of features that may be specific to particular implementations. Certain features that are described in this specification in the context of separate implementations can also be implemented in combination in a single implementation. Conversely, various features that are described in the context of a single implementation can also be implemented in multiple implementations separately or in combination with one another. Moreover, although features may be described herein as acting in certain combinations and even initially claimed as such, one or more features from a claimed combination can in some cases be excised from the combination, and the claimed combination may be directed to a sub-combination of features.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system modules and components in the implementations described herein should not be understood as requiring such separation in all implementations.

Particular implementations of the subject matter have been described. Other implementations are within the scope of the following claims.

## Claims

1. A syringe barrel (12) comprising
a hollow body with an opening (20) configured to receive a stopper (14),
a tip (18) configured for connection to a hollow needle (26), and
a compressible spacer (28) arranged inside the hollow body and adjacent to the tip (18), wherein the spacer (28) comprises an insoluble material configured to displace a liquid filled in the hollow body, **characterized in that**
there is a gap between the compressible spacer (28) and the inner surface of the barrel (12), and
the compressible spacer (28) is configured to displace but not to absorb or retain the liquid inside of the barrel (12).

2. The syringe barrel (12) of claim 1, further comprising a neck portion (38) arranged between the hollow body and the tip (18), wherein the spacer (28) comprises a protrusion (36), and wherein the neck portion (38) is configured to receive the protrusion (36).

3. The syringe barrel (12) of claim 1 or 2, wherein the hollow body is sized for a 0.5 ml, a 1.0 ml, or a 1.5 ml syringe (10), and the spacer (28) is configured to displace 10 to 100 µl, in particular 10 to 50 µl of a liquid.

4. The syringe barrel (12) of any of claims 1 to 3, wherein the spacer (28) consists of a single piece of material.

5. A syringe (10) comprising a syringe barrel (12) according to any one of claims 1 to 3 and a stopper (14) received in the body of the syringe barrel (12), with the spacer (28) arranged between the barrel tip (18) and the stopper (14).

6. The syringe (10) of claim 5, wherein the hollow body comprises an annular inner shoulder (32), wherein the spacer (28) rests against the annular inner shoulder (32), and an end face (34) of the stopper (14) abuts the spacer (28).

7. A method (100) of filling a syringe (10), comprising:
arranging (110) a syringe barrel (12) in a holder with an open end of the syringe barrel (12) pointing upwards;
inserting (120) a compressible spacer (28) that comprises an insoluble material configured to displace a liquid into the syringe barrel (12) through the open end;
filling (130) the syringe barrel (12) with the liquid, in particular a liquid pharmaceutical composition, through the open end; and
inserting (140) a stopper (14) into the syringe barrel (12) through the open end, **characterized in that**
there is a gap between the compressible spacer (28) and the inner surface of the barrel (12), and
the compressible spacer (28) is configured to displace but not to absorb or retain the liquid inside of the barrel (12).

8. The method (100) of claim 7, wherein the liquid pharmaceutical composition comprises vascular endothelial growth factor (VEGF) inhibitors.

9. The method (100) of claim 7 or 8, wherein
the syringe barrel (12) is sized for a 0.5 ml, a 1.0 ml, or a 1.5 ml syringe (10), and
filling (130) the syringe barrel (12) with a liquid comprises filling (130) 10 µl to 100 µl of the liquid into the syringe barrel (12).

10. The method (100) of any of claims 7 to 9, wherein inserting (120) the spacer (28) into the syringe barrel (12) through the open end comprises:
arranging a tube with one end inside of the syringe barrel (12);
inserting the spacer (28) into an opposite end of the tube; and
pushing, using a plunger, the spacer (28) through the tube and into the syringe barrel (12).

11. The method (100) of any of claims 7 to 10,
wherein the syringe barrel (12) is one of a plurality of syringe barrels (12) arranged in a tray, with the open end of each syringe barrel (12) pointing upwards,
wherein a compressible spacer (28) is inserted into each syringe barrel (12) in the tray,
further comprising sealing the tray for storage before filling (130) each syringe barrel (12) with the liquid.

12. The method (100) of any one of claims 7 to 11, comprising
using a filling and vent tube stoppering machine to fill multiple syringe barrels (12) with the liquid and insert stoppers (14) into multiple syringe barrels (12) in parallel.

## Patentansprüche

1. Spritzenzylinder (12), umfassend
einen Hohlkörper mit einer Öffnung (20), die zur Aufnahme eines Stopfens (14) ausgelegt ist,
eine Spitze (18), die zur Verbindung mit einer Hohlnadel (26) ausgelegt ist, und
einen komprimierbaren Abstandshalter (28), der in dem Hohlkörper und der Spitze (18) benachbart angeordnet ist, wobei der Abstandshalter (28) ein unlösliches Material umfasst, das dazu ausgelegt ist, eine in den Hohlkörper gefüllte Flüssigkeit zu verdrängen, **dadurch gekennzeichnet, dass**
sich zwischen dem komprimierbaren Abstandshalter (28) und der Innenfläche des Zylinders (12) ein Spalt befindet und der komprimierbare Abstandshalter (28) dazu ausgelegt ist, die Flüssigkeit in dem Zylinder (12) zu verdrängen, jedoch nicht zu absorbieren oder zurückzuhalten.

2. Spritzenzylinder (12) nach Anspruch 1, ferner umfassend einen Halsabschnitt (38), der zwischen dem Hohlkörper und der Spitze (18) angeordnet ist, wobei der Abstandshalter (28) einen Vorsprung (36) umfasst und wobei der Halsabschnitt (38) zur Aufnahme des Vorsprungs (36) ausgelegt ist.

3. Spritzenzylinder (12) nach Anspruch 1 oder 2, wobei der Hohlkörper für eine 0,5 ml-, eine 1,0 ml- oder eine 1,5 ml-Spritze (10) bemessen ist und der Abstandshalter (28) zum Verdrängen von 10 bis 100 µl, insbesondere 10 bis 50 µl einer Flüssigkeit ausgelegt ist.

4. Spritzenzylinder (12) nach einem der Ansprüche 1 bis 3, wobei der Abstandshalter (28) aus einem einzigen Materialstück besteht.

5. Spritze (10), umfassend einen Spritzenzylinder (12) nach einem der Ansprüche 1 bis 3 und einen Stopfen (14), der in dem Körper des Spritzenzylinders (12) aufgenommen ist, wobei der Abstandshalter (28) zwischen der Zylinderspitze (18) und dem Stopfen (14) angeordnet ist.

6. Spritze (10) nach Anspruch 5, wobei der Hohlkörper eine ringförmige innere Schulter (32) umfasst, wobei der Abstandshalter (28) an der ringförmigen inneren Schulter (32) anliegt und eine Endfläche (34) des Stopfens (14) an dem Abstandshalter (28) anliegt.

7. Verfahren (100) zum Füllen einer Spritze (10), umfassend:
Anordnen (110) eines Spritzenzylinders (12) in einem Halter, wobei ein offenes Ende des Spritzenzylinders (12) nach oben weist,
Einführen (120) eines komprimierbaren Abstandshalters (28), der ein unlösliches Material umfasst, das dazu ausgelegt ist, eine Flüssigkeit durch das offene Ende in den Spritzenzylinder (12) zu verdrängen,
Füllen (130) des Spritzenzylinders (12) mit der Flüssigkeit, insbesondere einer flüssigen pharmazeutischen Zusammensetzung, durch das offene Ende und
Einführen (140) eines Stopfens (14) in den Spritzenzylinder (12) durch das offene Ende, **dadurch gekennzeichnet, dass**
sich zwischen dem komprimierbaren Abstandshalter (28) und der Innenfläche des Zylinders (12) ein Spalt befindet und der komprimierbare Abstandshalter (28) dazu ausgelegt ist, die Flüssigkeit in dem Zylinder (12) zu verdrängen, jedoch nicht zu absorbieren oder zurückzuhalten.

8. Verfahren (100) nach Anspruch 7, wobei die flüssige pharmazeutische Zusammensetzung VEGF-Inhibitoren (VEGF = Vascular Endothelial Growth Factor) umfasst.

9. Verfahren (100) nach Anspruch 7 oder 8, wobei
der Spritzenzylinder (12) für eine 0,5 ml-, eine 1,0 ml- oder eine 1,5 ml-Spritze (10) bemessen ist und
das Füllen (130) des Spritzenzylinders (12) mit einer Flüssigkeit das Füllen (130) von 10 µl bis 100 µl der Flüssigkeit in den Spritzenzylinder (12) umfasst.

10. Verfahren (100) nach einem der Ansprüche 7 bis 9, wobei das Einführen (120) des Abstandshalters (28) in den Spritzenzylinder (12) durch das offene Ende Folgendes umfasst:
Anordnen einer Röhre mit einem Ende in dem Spritzenzylinder (12),
Einführen des Abstandshalters (28) in ein gegenüberliegendes Ende der Röhre und
Drücken des Abstandshalters (28) unter Verwendung eines Kolbens durch die Röhre und in den Spritzenzylinder (12).

11. Verfahren (100) nach einem der Ansprüche 7 bis 10, wobei der Spritzenzylinder (12) einer einer Vielzahl von Spritzenzylindern (12) ist, die in einer Schale angeordnet sind, wobei das offene Ende jedes Spritzenzylinders (12) nach oben weist,
wobei ein komprimierbarer Abstandshalter (28) in jeden Spritzenzylinder (12) in der Schale eingeführt wird, ferner umfassend Verschließen der Schale zur Lagerung, bevor jeder Spritzenzylinder (12) mit der Flüssigkeit gefüllt (130) wird.

12. Verfahren (100) nach einem der Ansprüche 7 bis 11, umfassend:
Verwenden einer Befüll- und Entlüftungsröhrenstopfmaschine, um mehrere Spritzenzylinder (12) mit der Flüssigkeit zu befüllen und Stopfen (14) parallel in mehrere Spritzenzylinder (12) einzuführen.

## Revendications

1. Cylindre de seringue (12) comprenant un corps creux avec une ouverture (20) configurée pour recevoir un bouchon (14),
un embout (18) configuré pour être relié à une aiguille creuse (26), et
un espaceur compressible (28) agencé à l'intérieur du corps creux et adjacent à l'embout (18), l'espaceur (28) comprenant un matériau insoluble configuré pour déplacer un liquide de remplissage dans le corps creux, **caractérisé en ce que**
il existe un espace entre l'espaceur compressible (28) et la surface intérieure du cylindre (12), et
l'espaceur compressible (28) est configuré pour déplacer mais non pour absorber ou retenir le liquide à l'intérieur du cylindre (12).

2. Cylindre de seringue (12) selon la revendication 1, comprenant en outre une partie de col (38) agencée entre le corps creux et l'embout (18), l'espaceur (28) comprenant une protubérance (36), et la partie de col (38) étant configurée pour recevoir la protubérance (36).

3. Cylindre de seringue (12) selon la revendication 1 ou 2, le corps creux étant dimensionné pour une seringue (10) de 0,5 ml, de 1,0 ml ou de 1,5 ml, et l'espaceur (28) étant configuré pour déplacer 10 à 100 µl, en particulier 10 à 50 µl d'un liquide.

4. Cylindre de seringue (12) selon l'une quelconque des revendications 1 à 3, l'espaceur (28) étant constitué d'une seule pièce de matériau.

5. Seringue (10) comprenant un cylindre de seringue (12) selon l'une quelconque des revendications 1 à 3 et un bouchon (14) reçu dans le corps du cylindre de seringue (12), l'espaceur (28) étant agencé entre l'embout de cylindre (18) et le bouchon (14).

6. Seringue (10) selon la revendication 5, le corps creux comprenant un épaulement intérieur annulaire (32), l'espaceur (28) reposant sur l'épaulement intérieur annulaire (32), et une face d'extrémité (34) du bouchon (14) venant en butée contre l'espaceur (28).

7. Procédé (100) de remplissage d'une seringue (10), comprenant :
l'agencement (110) d'un cylindre de seringue (12) dans un support avec une extrémité ouverte du cylindre de seringue (12) pointant vers le haut ;
l'insertion (120) d'un espaceur compressible (28) qui comprend un matériau insoluble configuré pour déplacer un liquide dans le cylindre de seringue (12) à travers l'extrémité ouverte ;
le remplissage (130) du cylindre de seringue (12) avec le liquide, en particulier une composition pharmaceutique liquide, par l'extrémité ouverte ; et
l'insertion (140) d'un bouchon (14) dans le cylindre de seringue (12) par l'extrémité ouverte, **caractérisé en ce que**
il existe un espace entre l'espaceur compressible (28) et la surface intérieure du cylindre (12), et
l'espaceur compressible (28) est configuré pour déplacer mais non pour absorber ou retenir le liquide à l'intérieur du cylindre (12).

8. Procédé (100) selon la revendication 7, la composition pharmaceutique liquide comprenant des inhibiteurs du facteur de croissance de l'endothélium vasculaire (VEGF).

9. Procédé (100) selon la revendication 7 ou 8,
le cylindre de seringue (12) étant dimensionné pour une seringue (10) de 0,5 ml, 1,0 ml ou 1,5 ml, et
le remplissage (130) du cylindre de seringue (12) avec un liquide comprenant le remplissage (130) de 10 µl à 100 µl du liquide dans le cylindre de seringue (12).

10. Procédé (100) selon l'une quelconque des revendications 7 à 9, l'insertion (120) de l'espaceur (28) dans le cylindre de seringue (12) par l'extrémité ouverte comprenant :
l'agencement d'un tube avec une extrémité à l'intérieur du cylindre de seringue (12) ;
l'insertion de l'espaceur (28) dans une extrémité opposée du tube ; et
la poussée, à l'aide d'un piston, de l'espaceur (28) à travers le tube et dans le cylindre de seringue (12).

11. Procédé (100) selon l'une quelconque des revendications 7 à 10,
le cylindre de seringue (12) faisant partie d'une pluralité de cylindres de seringue (12) agencés dans un plateau, l'extrémité ouverte de chaque cylindre de seringue (12) pointant vers le haut,
un espaceur compressible (28) étant inséré dans chaque cylindre de seringue (12) du plateau, comprenant en outre le scellement du plateau pour le stockage avant le remplissage (130) de chaque cylindre de seringue (12) avec le liquide.

12. Procédé (100) selon l'une quelconque des revendications 7 à 11, comprenant
l'utilisation d'une machine de remplissage et de bouchage de tubes d'aération pour remplir plusieurs cylindres de seringue (12) avec le liquide et insérer des bouchons (14) dans plusieurs cylindres de seringue (12) en parallèle.
